(19) Europäisches Patentamt European Patent Office Office européen des brevets

(11) **EP 4 411 747 A1**

(12) **EUROPEAN PATENT APPLICATION**

(43) Date of publication:
**07.08.2024 Bulletin 2024/32**

(21) Application number: **24155710.7**

(22) Date of filing: **05.02.2024**

(51) International Patent Classification (IPC):
**G16H 20/60** (2018.01)    **G16H 50/70** (2018.01)

(52) Cooperative Patent Classification (CPC):
**G16H 20/60; G16H 50/70;** G16H 50/20

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.02.2023 IT 202300001845**

(71) Applicant: **Wireless Sensor Networks S.r.L.**
**20900 Monza (MB) (IT)**

(72) Inventor: **CRESCINI, Matteo**
**20833 Giussano (MB) (IT)**

(74) Representative: **Rausa, Mario Alberto Manlio et al**
**Ing. Corradini & C. S.r.l.**
**Piazza Luigi di Savoia, 24**
**20124 Milano (IT)**

(54) **APPARATUS FOR MONITORING THE PARAMETERS OF A PLURALITY OF PATIENTS AND FOR PREDICTING THE TREND OF THE ADHERENCE BY THE PLURALITY OF PATIENTS TO A THERAPY**

(57)     An apparatus (1) for monitoring the parameters of a plurality of patients and for predicting the trend of the adherence by the plurality of patients to a therapy set by a specialist is described, said apparatus comprising:
- a plurality of devices (100) adapted to collect a plurality of health parameters of the plurality of patients relative to said therapy and to process health data in relation to said parameters,
- a central unit (500) adapted to receive health data of the plurality of patients from said plurality of devices; said central unit comprising
- a memory (25) in which a database (DB) containing said health data on the plurality of patients is installed,
- an artificial intelligence module (400) connected with said database and comprising at least one learning algorithm (401), said artificial intelligence module being adapted to determine the weight of the parameter and/or the weight of the patient for each parameter and patient of the previous plurality of patients as a function of the health data of the previous plurality of patients,
- a prediction module (501) connected with said database and configured to predict the trend of the adherence to said therapy by the current plurality of patients as a function of the health data contained in the database and as a function of the weight of the parameter and/or of the weight of the patient for each parameter and patient of the previous plurality of patients in output from the artificial intelligence module.

FIG.5

EP 4 411 747 A1

**Description**

TECHNICAL FIELD

The present invention concerns an apparatus for monitoring the parameters of a plurality of patients and for predicting the trend of the adherence by the plurality of patients to a therapy.

PRIOR ART

In recent years, the European healthcare system has experienced an increasing difficulty in finding resources to maintain a universal care model: in a finite market framework of resources and with a view to rationalizing health expenditure, many interventions have been aimed at introducing mechanisms that do not reconcile with the distinctive characteristics of a public service (fairness and equality of health care), resulting in the citizen's increasingly high co-participation in healthcare costs and at the same time, lengthening the waiting lists for services, without any priority determined by the severity of their condition (present and prospective).

In recent years, telemedicine which permits managing a patient's disease remotely (in particular, from their home) is becoming more and more established as a new organizational model. Telemedicine can offer a greater flexibility to address different health contexts and at the same time improve access and quality of the services, reducing costs (in the medium and long term especially for the National Health System) and the direct contribution of healthcare professionals (for example, minimizing the need for the patient to go in person to the clinic).

Unequal access to health care is a further (global) medical problem, which telemedicine can mitigate by improving the capacity, the quality and the efficiency of health care in disadvantaged areas and reducing the unequal distribution of medical resources, thereby helping to solve the difficulty and the high costs of accessing medical services.

The COVID-19 pandemic crisis, among many social, economic and health problems, has fortunately also brought new opportunities, such as the need for the patients and the healthcare professionals to look for new ways to stay healthy with minimal physical contact; with telemedicine, potentially risky events are detected and treated beforehand, and the number of unscheduled visits to the hospital can be significantly reduced, without this affecting the health-related quality of life of patients.

Telemedicine, however, requires flexibility and the involvement of a multidisciplinary team, which must respond to the problems of an increasing number of patients (and their caregivers) on an ongoing basis. Family caregivers of the people with chronic pathologies treated at home have a very demanding role in the management of their relatives, often experiencing a heavy care burden and this situation reduces their quality of life and increases their psychological distress and the risk of developing or aggravating health problems.

In addition, most telemedicine applications are developed for heterogeneous environments and, given their different nature, sharing and analysing data coming from dissimilar telemedicine applications is a difficult task for the specialist, who prescribes the treatment and who must remotely monitor multiple chronic patients, even simultaneously. A relevant management problem is poor adherence to the therapy (and therefore, to the pharmacological and not pharmacological treatments prescribed by the specialist): clinical studies indicate that home care monitoring can be effective, but that the results may not materialize in practice because many patients have adherence problems and, despite the progresses in the therapy, the situation remains challenging overall. Numerous studies have in fact reported low adherence and persistence to therapy, which acts as a barrier to the control and the successful management of the chronic patient at his or her home.

With the emergence of a connected ecosystem, it is thus moved on to the provision of advanced personalized therapies, which have the potential of improving one's and the clinical self-management. Over time, the improvements can combine the therapy data with the previously missing contextualized data of the patient (including, for example, meal and activity data), to support personalized clinical decisions, and ultimately to revolutionize therapy management.

An object of the present invention is to make available a tele-monitoring apparatus, as a means of communication between patients, doctors and home care service providers, to support the patients clinically and technically and to improve the adherence to the therapy in patients with chronic diseases.

Such object is achieved by the features of the invention reported in the independent claim. The dependent claims outline preferred and/or particularly advantageous aspects of the invention.

DISCLOSURE OF THE INVENTION

In particular, the invention makes available an apparatus for monitoring the parameters of a plurality of patients and for predicting the trend of adherence by the plurality of patients to a therapy set by a specialist is described, said apparatus comprising:

- a plurality of devices adapted to collect parameters on the health of the plurality of patients relative to said therapy and to process health data in relation to said parameters,
- a central unit adapted to receive health data of the plurality of patients from said plurality of devices; said central unit comprising
- a memory in which a database containing said health data on the plurality of patients is installed,
- an artificial intelligence module (400) connected with said database and comprising at least one learning algorithm, said artificial intelligence module being adapted to determine the weight of the parameter and/or the weight of the patient for each parameter and patient of the previous plurality of patients as a function of the health data of the previous plurality of patients,
- a prediction module connected with said database and configured to predict the trend of the adherence to said therapy by the current plurality of patients as a function of the health data contained in the database and as a function of the weight of the parameter and/or of the weight of the patient for each parameter and patient of the previous plurality of patients in output from the artificial intelligence module.

The present invention therefore aims to optimize the taking charge of the person suffering from or at risk of developing a chronic disease, providing the specialist in charge with a smart tool for prioritizing care interventions, so that he or she knows (with increasingly educated responses from constant learning) which patient to prioritise for intervention, with the same level of resources, and which vital parameter to prioritise for monitoring, for each patient thus selected.

The patient is no longer a passive subject but an active player in this process, but care self-management is not the subject therein, which instead revolves around the objectification of the adherence to the therapy, by parameter and by patient, by means of an innovative AI-based system.

The foundation of the invention (programming of the taking charge and corollary for the evaluation of the effectiveness thereof), is the learning that derives from the data coming from the information, aggregate and analytical flows of the NHS; the system reconstructs the determinants of health, in terms of therapeutic adherence and outcomes, through an innovative artificial intelligence algorithm.

BRIEF DESCRIPTION OF THE DRAWINGS

Further features and advantages of the invention will be more apparent after reading the following description provided by way of a non-limiting example, with the aid of the figures illustrated in the accompanying tables.

Figure 1 is a schematic view of a device of the apparatus for monitoring the parameters of a plurality of patients and for predicting the trend of adherence by the plurality of patients to a therapy in accordance with the present invention.
Figure 2 shows the fuzzy logic of the device of figure 1.
Figure 3 is a schematic view of the apparatus in accordance with an embodiment of the invention;
Figure 4 is a diagram of the Artificial Intelligence module;

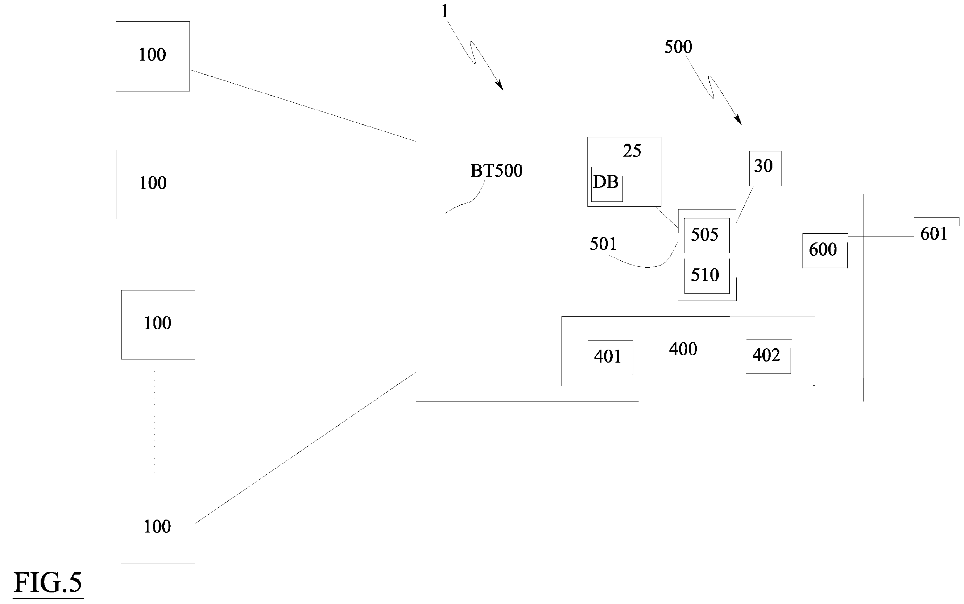

Figure 5 is a schematic view of the apparatus in accordance with a variant of the embodiment of the invention.

BEST MODE OF THE INVENTION

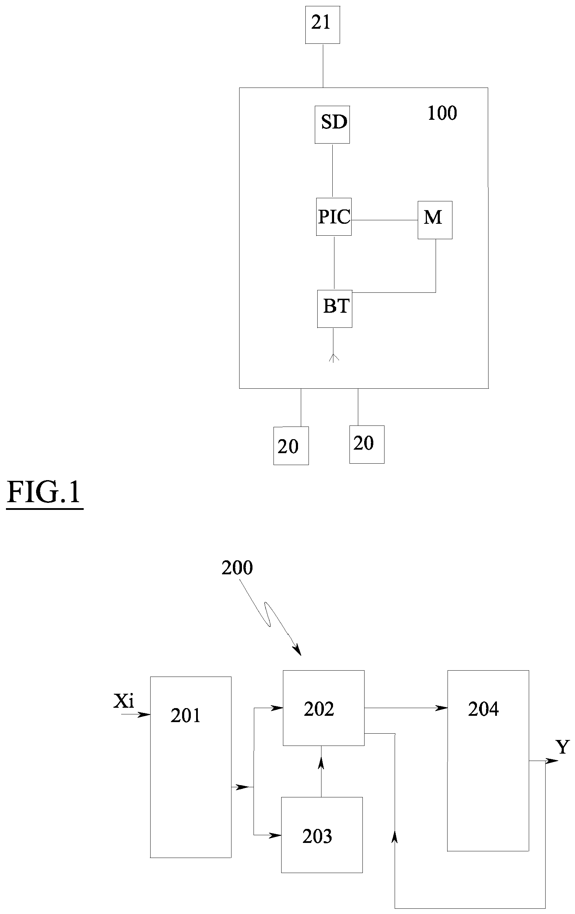

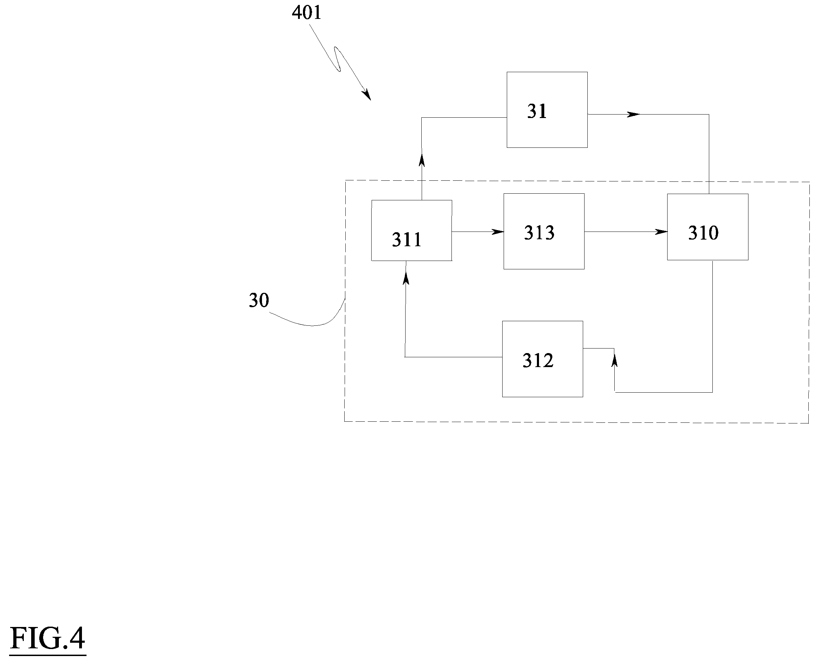

With particular reference to Figures 1-4, the apparatus for monitoring the parameters of a plurality of patients and for predicting the trend of adherence by the plurality of patients to a therapy in accordance with the present invention has been indicated overall with 1. The plurality of patients who have in common, alternatively, the same chronic pathology and/or comparable degrees of comorbidity (for example: the patients may suffer from heart failure and therefore have in common the same chronic pathology, or suffer from both hypertension and Alzheimer's and therefore have comparable degrees of comorbidity).

The apparatus 1 comprises means adapted to collect parameters on the health of the plurality of patients relative to the therapy of the chronic patient.

The parameters on the health of the patient may concern vital parameters of each patient such as, for example, Systolic Arterial Pressure, normally expressed in mmHg, and/or the patient's heart rate normally expressed in *bpm* (i.e., beats per minute).

The parameters can also concern the patient's diet, or the state of pain perceived by the patient, and so on.

The means comprise at least one device 100, preferably an interface, for example a local computer, a tablet or a smartphone connectable to a central unit 500, i.e. a dedicated web server 500, via the web via cable or wireless preferably through an HTTPS connection. Preferably the means comprise a plurality of devices 100, one for each patient.

The device 100 can be connected to sensors 20 that detect vital parameters of the patient, for example Systolic Arterial Pressure and/or Heart Rate, and/or it is possible that the device 100 is equipped with input means 21, for example a keyboard or the like, for the manual entry of the parameters on the patient by a caregiver or a user or the patient him- or herself, such entry being possible also thanks to a known technology that can allow the recognition and the translation of the spoken language into written text by the local computer.

Each device 100 communicates with a dedicated web server 500, preferably in HTTPS mode.

The device 100 comprises a control unit comprising an electronic card in which there is mounted a PIC microprocessor, a removable SD memory card and preferably a BT data transmission/reception interface in RF radio frequency, preferably of the wireless type, and preferably compatible for the transmission/reception of data through Bluetooth protocol and/or connected to a modem M provided with a SIM card for wireless radio frequency connection. The device 100 is powered externally, preferably by means of a power supply with wall outlet, but it is also possible to power it with a battery (so-called external "power-bank").

The control unit preferably comprises an executive software FL that operates in accordance with a fuzzy logic by implementing a fuzzy logic controller 200 shown in Figure 2. The controller 200 comprises a fuzzification interface or fuzzifier 201 that transforms the sharply measured parameters into adequate linguistic values, by following a fuzzification procedure that transforms objective data into subjective data through a mapping of the inputs in fuzzy set labels in each specific reference universe, converting each value $x_{i,j}$ of the input data or parameter ( $x_{i,j}^{t_z}$ represents the value of the $j$ - $th$ parameter for the $i$ - $th$ patient) at the time $t_z$, for $z = 0, ..., k$ (the sampling time $t_z$ depends on the type of data or $j$ - $th$ parameter or on the type of $i$ - $th$ patient):

$$x_{i,j}^{t_z},$$

in a single pair (a *fuzzy set*) wherein the belonging of $x_{i,j}^{t_z}$ to the set can take all the values comprised in the interval [0,1]:

$$\left(x_{i,j}^{t_z}, \mu_i\left(x_{i,j}^{t_z}\right)\right).$$

The $\mu$ represents the *degree of belonging of the predicate* to the fuzzy set considered and consists of a real number included *between* 0 *and* 1, so that it is possible to consider a certain value not as belonging exclusively to a single set, but as belonging simultaneously, although to a different extent, to several distinct sets.

The basic fuzzy control rules are characterized by a collection of fuzzy rules $IF \rightarrow THEN$ in which the (antecedent) preconditions and the consequent ones involve variables, according to this form:

$R^i$: IF x is $A_i$, ...,AND y is $B_i$, THEN z is $C_i$ i = 1 ... n. where x, ..., y and z are variables representing respectively the process state variables and the control variable and $A_i$, ..., $B_i$, $C_i$, are the values of the variables $x$, ..., $y$ and $z$.

The controller 200 comprises the inference engine 202 and a database comprises the basic rules 203. The inference engine 202 has the task of calculating the membership functions as a function of the variables in input to the fuzzifier 201 and as a function of the basic rules 203. In addition, the controller 200 is of the closed-loop type in that the membership functions are also calculated as a function of the results of the previous cases. The controller 200 also comprises a defuzzer 204 adapted to convert the linguistic values in output into data, in particular into the signal $y$.

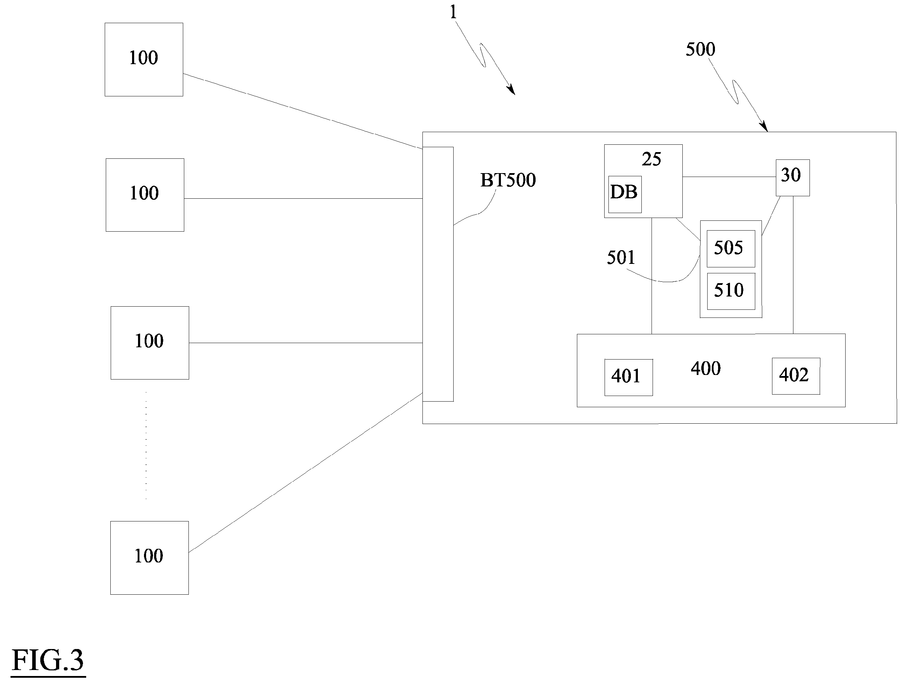

Preferably the device 100 belongs to a plurality of devices 100 connected with the web server 500 preferably wirelessly via the interfaces BT of the single devices 100 and the interface BT500 of the web server 500, as visible in figure 3.

Preferably, data processing also takes place in a dedicated partition of the web server 500, where the basic rules and the inference engine integrated in each device 100 locally are replicated and reside: in this case the system behaves as an apparatus for the indirect monitoring of the patient's state and the use of the controller 200 allows to manage this task following a continuous process of acquisition of the inputs and of processing of the output, for all the devices 100 connected in the network. Consequently, all network-connected devices 100 can draw on these resources in the fuzzification and defuzzification process, by connecting to the aforesaid web server via a connection preferably of the HTTPS type.

In the connection, each device 100 feeds an existing database with the value updated to the latest detection of each parameter that defines the single rules, which are thus updated continuously and over time, for each device 100 in the network. At each connection the value of each parameter is made available for download, to each device 100 connected in the network. The aforesaid upload and/or download connection to the web server, preferably of the HTTPS type, is guaranteed by the use of a gateway (to which the device 100 connects wirelessly via a compatible Bluetooth interface) or via the modem M with integrated SIM. Thus, upon installation of each device 100, this, by connecting to such web server 500 can download the value of the parameters of the updated rules, without starting from the initial value defined by the single rule.

The device 100 or the web server 500 acquires the value of the signal $x_{i,j}^{t_z}$ referred to the $i$ - $th$ patient.

$$x_{i,j}^{t_z}$$

with: $i$ = 1, ... , $m$; $j$ = 1, ..., $n$; $z$ = 1, ..., $k$; $x_{i,j}^{t_z}$ represents the $j$ - $th$ value of the parameter $x$ for each $i$ - $th$ patient acquired by the web server 500 at the time $t_z$, with a sampling $z$ that depends on the type of parameter and/or on the type of patient and/or on the pathology; since each device 100 corresponds to a patient, the device 100 acquires the value of the signal $x_j^{t_z}$ . As already stated, the parameter $x_{i,j}^{t_z}$ can also consists of a subjective element, provided that this value can be expressed in cardinal terms. For example: the parameter $j$ of $x_{i,j}^{t_z}$ can refer to the pain perceived by the patient, if this parameter is expressed with cardinal numbers, for example, on a scale from 1 to 10.

Another input value is the *target value* of the $j$ - $th$ signal for the $i$ - $th$ patient, established by the user (preferably the specialist who treats the patient), that is the parameter:

$$m_{i,j}^{t_z}$$

with: $i$ = 1, ..., $m$; $j$ = 1, ...,$n$ ; $z$ = 1, ..., $k$

Another input value is the *variation of the signal with respect to the target value* $m_{i,j}^{t_z}$ , the parameter:

$$\sigma_{i,j}^{t_z}$$

with: $i$ = 1, ..., $m$; $j$ = 1, ...,$n$ ; $z$ = 1, ..., $k$ which represents the variation (+), at the time $t_z$, of the value of $x_{i,j}^{t_z}$ for each $j$ - $th$ parameter and $i$ - $th$ patient, by $x_{i,j}^{t_z}$ with respect to the target value $m_{i,j}^{t_z}$ :

$$\sigma_{i,j}^{t_z} = (x_{i,j}^{t_z} \mp m_{i,j}^{t_z}).$$

The entry of the target value and of the variation of the signal with respect to the target value are normally carried out by the user (preferably the specialist who treats the patient) via the keyboard 21 and are the expression of the therapy set for the patients.

Each device 100 or the web server 500 calculates the membership function for the input parameters for each patient via the controller 200:

$$\mu\left(x_{i,j}^{t_z}\right)$$

The membership functions are different for each type of $j$ - $th$ parameter and as a function of the $i$ - $th$ patient ( $x_i^{t_z}$ ) and the value $y$ in output from the controller 200 of each device 100 is the membership function $\mu(x_{i,j}^{t_z})$ .

As mentioned, the sampling time $t_z$ depends on the *type of signal* (the *parameter $x_j$*) or on the *type of patient* ($x_i$).

The $j$ - $th$ parameters can be acquired in two ways:

- *automatically,* in case the value of the $j$ - $th$ parameter is automatically transmitted by a sensor or medical detection device, connected with the device 100 for example via an integrated compatible Bluetooth module;

or:

- *manually,* in case the value of the $j$ - $th$ parameter is entered manually by the user, for example, via the keyboard 21.

In addition, the $j$ - $th$ parameter can be expressed, natively:

- by means of *a real number,* and therefore be natively an "objective" value

or:

- through *a subjective judgement,* and thus be natively a "subjective" value.

Below are the different membership functions for some of the different parameters acquired by the devices 100: body temperature ( $x_{i,t}^{t_z}$ ); heart rate ( $x_{i,f}^{t_z}$ ); functional oxygen saturation ( $x_{i,s}^{t_z}$ ); body mass index ( $x_{i,b}^{t_z}$ ); blood pressure ( $x_{i,p}^{t_z}$ ); subjective perception of pain ( $x_{i,d}^{t_z}$ ); degree of urination ( $x_{i,w}^{t_z}$ ). For the different parameters acquired by the devices 100, an ideal value $m_{i,j}^{t_z}$ and a variance $\sigma_{i,j}^{t_z}$ are fixed which represent the reference parameters of the therapy set for the plurality of patients.

For example, the apparatus acquires the $j$ - $th$ vital parameter: *"body temperature",* ( $x_{i,t}^{t_z}$ ).

The $j$ - $th$ parameter *"body temperature"* ( $x_{i,t}^{t_z}$ ) is an example of a vital parameter for which it is possible to set a maximum reference threshold and/or a minimum reference threshold; it is in fact possible for the user (preferably the specialist who prescribes home care) to fix a *"minimum"* body temperature threshold and a *"maximum"* body temperature threshold, respectively, below and above which he or she (the user) considers the trend of this $j$ - $th$ vital parameter (specifically *"t"*) for the $i$ - $th$ patient to be pathological: $x_{i,t}^{t_z}$ . For the representation of the membership function of the vital parameter *"body temperature"* ( $x_{i,t}^{t_z}$ ), a Gaussian curve is adopted:

$$f\left(x_{i,t}^{t_z}\right) = \frac{1}{\sqrt{2\pi\left(\sigma_{i,t}^{t_z}\right)^2}} \cdot e^{-\frac{1}{2}\cdot\frac{\left(x_{i,t}^{t_z}-m_{i,t}^{t_z}\right)^2}{\left(\sigma_{i,t}^{t_z}\right)^2}} .$$

For:

$$\frac{1}{\sqrt{2\pi\left(\sigma_{i,t}^{t_z}\right)^2}} = 1$$

the membership function, $\mu\left(x_{i,t}^{t_z}\right)$, is represented as follows:

$$\mu\left(x_{i,t}^{t_z}\right) = e^{-\frac{1}{2}\cdot\left(\frac{x_{i,t}^{t_z}-m_{i,t}^{t_z}}{\sigma_{i,t}^{t_z}}\right)^2}$$

The membership function follows an exponential type curve, which is continuous around the ideal value ( $m_{i,t}^{t_z}$ ) [at which $\mu\left(x_{i,t}^{t_z}\right) = 1$ ], symmetrical with respect to this abscissa and characterized by a second parameter ( $\sigma_{i,t}^{t_z}$ ) that expresses its width.

The area subtended to the curve is worth one and represents the sum of the probability of all the values of $\mu(x_{i,t}^{t_z})$ :

For each $x_{i,t}^{t_z}$ the specialist therefore fixes an ideal value for:

- $m_{i,t}^{t_z}$, relative to the "$j$ - $th$" specific parameter (specifically "$t$"), for each "$i$ - $th$" specific patient;

- $\sigma_{i,t}^{t_z}$, relative to the variation $\pm$ with respect to $m_{i,t}^{t_z}$.

The membership function of the $j$ - $th$ vital parameter "body temperature" ( $x_{i,t}^{t_z}$ ) is:

$$\mu\left(x_{i,t}^{t_z}\right) = \begin{cases} e^{-\frac{1}{2}\cdot\left(\frac{x_{i,t}^{t_z}-m_{i,t}^{t_z}}{\sigma_{i,t}^{t_z}}\right)^2}, if\ (m_{i,t}^{t_z} - \sigma_{i,t}^{t_z}) \leq x_{i,t}^{t_z} \leq (m_{i,t}^{t_z} + \sigma_{i,t}^{t_z})\ and\ \sigma_{i,t}^{t_z} \neq 0; \\ 0, if\ x_{i,t}^{t_z} < (m_{i,t}^{t_z} - \sigma_{i,t}^{t_z})\ or\ x_{i,t}^{t_z} > (m_{i,t}^{t_z} + \sigma_{i,t}^{t_z}); \\ 1, if\ x_{i,t}^{t_z} = m_{i,t}^{t_z} \end{cases}$$

The rule of the membership function is:

- IF $\mu\left(x_{i,t}^{t_z}\right) < \left(m_{i,t}^{t_z} - \sigma_{i,t}^{t_z}\right)$ THEN 4 the reference parameter is *below the minimum pathological value* (or *TOO LOW* or *PATHOLOGICAL*).

- IF $\mu\left(x_{i,t}^{t_z}\right) > \left(m_{i,t}^{t_z} + \sigma_{i,t}^{t_z}\right)$ THEN 4 the reference parameter is *above the maximum pathological value* (or *TOO HIGH* or *PATHOLOGICAL*).

An adult's normal body temperature can vary indicatively from 35.2°C ÷ 36.9°C; after which we have a light fever, which becomes high starting from 38°C and very high when it reaches 39,5°C or 41 °C.

Consequently, in the case of the *j - th* parameter *"body temperature"* ( $x_{i,t}^{t_z}$ ) the pathological thresholds are:

| | | |
|---|---|---|
| $m_{i,t}^{t_z}$ | *Ideal* value of body temperature: | 36°C. |
| $\left(m_{i,t}^{t_z} - \sigma_{i,t}^{t_z}\right)$ | *Minimum pathological* value of body temperature: | 34°C. |
| $\left(m_{i,t}^{t_z} + \sigma_{i,t}^{t_z}\right)$ | *Maximum pathological* value of body temperature: | 38°C. |

The apparatus also acquires the *j - th* vital parameter: *"heart rate"*.

Like for the *j - th* parameter *"body temperature"*, the vital parameter *"heart rate"* ( $x_{i,f}^{t_z}$ ) is also an example of a parameter for which it is possible to set a maximum and/or minimum reference threshold, for which a *"minimum"* heart rate threshold and a *"maximum"* heart rate threshold can be fixed, respectively below and above which the user (preferably the specialist who prescribes home care) considers the trend of this *j - th* vital parameter for the *i - th* patient to be pathological.

For the representation of the membership function of the vital parameter *"heart rate"*, a Gaussian curve is adopted.

The membership function is:

$$\mu\left(x_{i,f}^{t_z}\right) = \begin{cases} e^{-\frac{1}{2}\cdot\left(\frac{x_{i,f}^{t_z}-m_{i,f}^{t_z}}{\sigma_{i,f}^{t_z}}\right)^2}, se\ \left(m_{i,f}^{t_z} - \sigma_{i,f}^{t_z}\right) \leq x_{i,f}^{t_z} \leq \left(m_{i,f}^{t_z} + \sigma_{i,f}^{t_z}\right)\ and\ \sigma_{i,f}^{t_z} \neq 0; \\ 0, if\ x_{i,f}^{t_z} < \left(m_{i,f}^{t_z} - \sigma_{i,f}^{t_z}\right)\ or\ x_{i,f}^{t_z} > \left(m_{i,f}^{t_z} + \sigma_{i,f}^{t_z}\right); \\ 1, if\ x_{i,f}^{t_z} = m_{i,f}^{t_z} \end{cases}$$

The rule of the membership function is:

- IF $\mu\left(x_{i,f}^{t_z}\right) < \left(m_{i,f}^{t_z} - \sigma_{i,f}^{t_z}\right)$ THEN 4 the reference parameter is *below the minimum pathological value* (or *TOO LOW* or *PATHOLOGICAL*).

- IF $\mu\left(x_{i,f}^{t_z}\right) > \left(m_{i,f}^{t_z} + \sigma_{i,f}^{t_z}\right)$ THEN 4 the reference parameter is *above the maximum pathological value* (or *TOO HIGH* or *PATHOLOGICAL*).

In resting condition the rate of the heartbeat is regular and, generally, is between 60 and 100 beats per minute (*bpm*). It is called tachycardia when the heart rate is higher than 100 *bpm*.

Consequently, in the case of the *i - th* parameter *"heart rate"* ( $x_{i,f}^{t_z}$ ), the pathological values are:

| | | |
|---|---|---|
| $m_{i,f}^{t_z}$ | *Ideal* value of heart rate: | 70 bpm. |
| $(m_{i,f}^{t_z} - \sigma_{i,f}^{t_z})$ | *Minimum pathological* value of heart rate | 50 bpm. |
| $(m_{i,f}^{t_z} + \sigma_{i,f}^{t_z})$ | *Maximum pathological* value of heart rate | 90 bpm. |

The apparatus also detects the vital parameter *"functional oxygen saturation"*.

The *j - th* parameter *"functional oxygen saturation"* (abbreviated to: S$_P$O$_2$%) ( $x_{i,s}^{t_z}$ ) is an example of a parameter for which it is possible to set a minimum reference threshold, for which a threshold of *"minimum"* S$_P$O$_2$% can be fixed, while the maximum threshold is the reference "Full Scale" of the vital parameter (100%); only below the minimum threshold the user (preferably the specialist who prescribes home care) considers the trend of the parameter to be pathological, while the value of the parameter cannot go beyond the maximum threshold.

For the representation of the membership function of the vital parameter *"functional oxygen saturation"*, a Gaussian curve is adopted.

The membership function of the *j - th* parameter *"functional oxygen saturation"* (S$_P$O$_2$%) is:

$$\mu\left(x_{i,s}^{t_z}\right) = \begin{cases} e^{-\frac{1}{2}\cdot\left(\frac{x_{i,s}^{t_z}-m_{i,s}^{t_z}}{\sigma_{i,s}^{t_z}}\right)^2}, if\ (m_{i,s}^{t_z} - \sigma_{i,s}^{t_z}) \leq x_{i,s}^{t_z}\ and\ \sigma_{i,s}^{t_z} \neq 0; \\ 0, if\ x_{i,s}^{t_z} < \left(m_{i,s}^{t_z} - \sigma_{i,s}^{t_z}\right); \\ 1, if\ x_{i,s}^{t_z} = m_{i,s}^{t_z} \end{cases}$$

The rule of the membership function of the *j - th* parameter *"functional oxygen saturation"* (S$_P$O$_2$%) is:

- IF $\mu\left(x_{i,s}^{t_z}\right) < (m_{i,s}^{t_z} - \sigma_{i,s}^{t_z})$ THEN 4 the reference parameter is *below the minimum pathological value* (or *TOO LOW* or PATHOLOGICAL).

The normal values detected with the pulse oximeter typically range between 95 and 100%. Values below 90% are considered low; consequently, in the case of the *j - th* parameter *"functional oxygen saturation"* the pathological threshold is:

| | | |
|---|---|---|
| $m_{i,s}^{t_z}$ | *Ideal* value of S$_P$O$_2$%: | 95÷99% |
| $(m_{i,s}^{t_z} - \sigma_{i,s}^{t_z})$ | *Minimum pathological* value of S$_P$O$_2$%: | 90% |

The apparatus also detects the vital parameter *"body mass index"* or *"BMI"*.

The vital parameter *"Body Mass Index"* also abbreviated to *"BMI"* ( $x_{i,b}^{t_z}$ ) represents an example of a parameter for which it is possible to set a maximum reference threshold; for example, this applies to *i - th* patients suffering from obesity and for which a *"maximum"* body weight threshold can be fixed, above which the user (preferably the specialist who prescribes home care) considers the trend of the parameter to be pathological.

For the representation of the membership function of the vital parameter *"BMI"*, a Gaussian curve is adopted.

The membership function of the *j - th* parameter *"BMI"* is:

$$\mu\left(x_{i,b}^{t_z}\right) = \begin{cases} e^{-\frac{1}{2}\cdot\left(\frac{x_{i,b}^{t_z}-m_{i,b}^{t_z}}{\sigma_{i,b}^{t_z}}\right)^2}, & if\ x_{i,b}^{t_z} \le (m_{i,b}^{t_z} + \sigma_{i,b}^{t_z})\ and\ \sigma_{i,b}^{t_z} \ne 0; \\ 0, & if\ x_{i,b}^{t_z} > (m_{i,b}^{t_z} + \sigma_{i,b}^{t_z}); \\ 1, & if\ x_{i,b}^{t_z} = m_{i,b}^{t_z} \end{cases}$$

The rule of the membership function of the *j - th* parameter *"BMI"* ( $x_{i,b}^{t_z}$ ) is:

- IF $\mu\left(x_{i,b}^{t_z}\right) < (m_{i,b}^{t_z} + \sigma_{i,b}^{t_z})$ THEN 4 the reference parameter is *above the maximum pathological value* (or *TOO HIGH or PATHOLOGICAL).*

According to the World Health Organization (WHO), the BMI threshold value in adults is 25 for overweight and 30 for obesity. The normal weight is indicated by a BMI between 18.5 and 24.9; consequently, in the case of the *j - th* parameter *"BMI"* the pathological threshold is:

| $m_{i,b}^{t_z}$ | *Ideal* value of BMI: | 20. |
|---|---|---|
| $(m_{i,b}^{t_z} + \sigma_{i,b}^{t_z})$ | *Maximum pathological* value of BMI: | 30. |

The apparatus also acquires the *i - th* vital parameter: *"diastolic* and/or *systolic blood pressure",* abbreviated to *"blood pressure".*

The *j - th* parameter *"blood pressure"* ( $x_{i,p}^{t_z}$ ), like for the "body temperature" parameter, represents a parameter for which a maximum and/or a minimum reference threshold can be set, for which a *"minimum"* "blood pressure threshold and a *"maximum"* blood pressure threshold can be fixed, respectively below and above which the user (preferably the specialist who prescribes home care) considers the trend of this *j - th* vital parameter to be pathological for the *i - th* patient and this applies to both *"diastolic pressure",* and to *"systolic pressure".*

For the representation of the membership function of the vital parameter *"blood pressure",* a Gaussian curve is adopted.

The membership function of the *j - th* vital parameter *"blood pressure"* ( $x_{i,p}^{t_z}$ ) is:

$$\mu\left(x_{i,p}^{t_z}\right) = \begin{cases} e^{-\frac{1}{2}\cdot\left(\frac{x_{i,p}^{t_z}-m_{i,p}^{t_z}}{\sigma_{i,p}^{t_z}}\right)^2}, & if\ (m_{i,p}^{t_z} - \sigma_{i,p}^{t_z}) \le x_{i,p}^{t_z} \le (m_{i,p}^{t_z} + \sigma_{i,p}^{t_z})\ and\ \sigma_{i,p}^{t_z} \ne 0; \\ 0, & if\ x_{i,p}^{t_z} < (m_{i,p}^{t_z} - \sigma_{i,p}^{t_z})\ or\ x_{i,p}^{t_z} > (m_{i,p}^{t_z} + \sigma_{i,p}^{t_z}); \\ 1, & if\ x_{i,p}^{t_z} = m_{i,p}^{t_z} \end{cases}$$

The rule of the membership function of the *i - th* vital parameter *"blood pressure"* ( $x_{i,p}^{t_z}$ ) is:

- IF $\mu\left(x_{i,p}^{t_z}\right) < (m_{i,p}^{t_z} - \sigma_{i,p}^{t_z})$ THEN → the reference parameter is *below the minimum pathological value* (or *TOO LOW or PATHOLOGICAL).*

- IF $\mu\left(x_{i,p}^{t_z}\right) > (m_{i,p}^{t_z} + \sigma_{i,p}^{t_z})$ THEN → the reference parameter is *above the maximum pathological value* (or *TOO HIGH* or PATHOLOGICAL).

There are values of maximum (also, systolic) and minimum (also, diastolic) blood pressure that are considered normal and recognized and shared by all the main medical-scientific institutes that deal with pressure: in general, experts agree with the definition of the hypertension as the situation in which the values of the systolic pressure (the "maximum" one) are higher than 140 mm Hg and those of the diastolic pressure (the "minimum" one) are higher than 90 mm Hg.

Consequently, in the case of the *j - th* parameter *"blood pressure"* of the *i - th* vital parameter *"blood pressure"* ( $x_{i,p}^{t_z}$ ), the pathological values are therefore:

| $m_{i,p}^{t_z}$ | *Ideal* value of the pressure (diastolic/systolic): | 75/115 mmHg |
|---|---|---|
| $(m_{i,p}^{t_z} - \sigma_{i,p}^{t_z})$ | *Minimum pathological* value of the pressure (diastolic/systolic): | 60/90 mmHg |
| $(m_{i,p}^{t_z} + \sigma_{i,p}^{t_z})$ | *Maximum pathological* value of the pressure (diastolic/systolic): | 90/140 mmHg |

The apparatus also acquires the *j - th* parameter *"pain dimensional scale"* ( $x_{i,d}^{t_z}$ ).

The parameter *"pain dimensional scale"* of the *i - th* patient represents an example of a parameter for which reference thresholds can be set to fixed values and for which the reference threshold is on a dimensional scale, for example, from number 1 to number 10, which expresses the level of pain perceived in numerical terms, from the lowest value to the highest value.

In the case of the fixed value parameter, like in the case of the *i - th* parameter *"pain dimensional scale"*, the membership function is:

$$\mu\left(x_{i,d}^{t_z}\right) = position \cdot \frac{100}{number\ of\ positions}\%$$

The rule of the membership function, in case of fixed value parameters is:

- IF $x_{i,d}^{t_z} > x_{i,d}^{t_z-1}$ THEN → the *situation of the patient* relatively to the parameter has *improved.*

- IF $x_{i,d}^{t_z} = x_{i,d}^{t_z-1}$ THEN → the *situation of the patient* relatively to the parameter has *not changed.*

- IF $x_{i,d}^{t_z} < x_{i,d}^{t_z-1}$ THEN → the *situation of the patient* relatively to the parameter has *worsened.*

The apparatus also detects subjective parameters, which depend on the manual entry (of the value) by the user, such as for example the *"degree of urination"* of the aid (i.e. of a diaper) worn by the patient.

An example of a parameter for which it is possible to set a value entry, acquired by the apparatus through manual entry is the parameter "the patient's diaper is wet" ( $x_{i,w}^{t_z}$ ), for which the answer to the question is given by the user manually and can be *"YES"* or *"NO"*. In the case of manual entry of the parameter, the membership function is linked to an *"affirmation"*, which can be true or false:

$$\mu\left(x_{i,w}^{t_z}\right) = \begin{cases} 1, if\ the\ affirmation\ is\ true \\ 0, otherwise. \end{cases}$$

The devices 100 may acquire all or only some of the parameters listed above.

All health data y in output from the devices 100, i.e. the various membership functions, and in input to the web server 500 are collected in a database DB.

The web server 500 comprises a processor 30_and a memory 25, containing the database DB in which the data on the health of each patient, i.e. the data received from the plurality of devices 100, are collected. Preferably in the memory 25, preferably in the database DB, the parameters used to determine the health data and preferably the patients are also stored.

The web server 500 comprises an artificial intelligence module 400 comprising a machine learning algorithm 401 adapted to receive the health data contained in the database DB. In particular, the learning algorithm 401 is adapted to receive the health data contained in the database DB and relative not to the current plurality of patients but to the previous plurality of patients.

The learning algorithm 401 is adapted to encode the methods for processing the clinical data of the patients with chronic pathology, to determine the weight of the parameter and/or the weight of the patient for each parameter and/or patient of the previous plurality of patients that feeds said database DB.

Preferably the module 401 contains a reinforcement module 30 and a control module 31. In learning by reinforcement, the module 30 adopts a policy gradient method, with the task of analysing the data and building the analytical model automatically, and it contains a processor that fits a learning module whose logic is based on the trial-and-error approach used by the specialists to select (among many) the patient to be followed with priority and/or the vital parameter to be measured (among many) and to be given priority over the others.

The control module 31 integrates a process controller whose algorithm uses a reward and penalty paradigm structured in sub-modules: an agent module 310; an environment module 311; an action module 312; a reward module 313; these modules develop their functionality using discrete time steps and through trials and errors, communicating seam-lessly preferably according to an *if-then* type communication logic.

During learning, the module 401 maps the inputs with the possible results, rewarding the desired behaviours, allowing the best outcomes (in terms of period compendium per parameter/patient) to be given greater weight, preferably using the following process: it performs operations within the environment module 311 and takes note of the new status and of the value of the reward 313 (preferably, the period compendium value per parameter and/or per patient); it associates the transition between operation-state module 31 to the value of the reward module 313. Once the model is completed, the agent module 310 simulates sequences of operations basing itself on the probability of optimal cumulative rewards. It then assigns new values to these sequences of operations via the action module 312. At this point, the agent 310 develops various strategies within the environment 311 to reach the period value of the weight of the parameter and/or of the weight of the patient, for each parameter and/or patient of the previous plurality of patients that feeds said database.

The architecture of the module 400 provides that given a set *mxm,* of *i* patients (with *i* = 1, ..., *m*) whose parameters *j* (with *j* = 1, ..., *n*), are measured, a learning algorithm 401 (with duration $n^m$ periods) is applied to determine the contribution of each parameter to the general state of the patient (the contribution or weight of the patient, $w_{i,j}^{t_z}$ , and the contribution or weight of the parameter or signal, $p_{i,j}^{t_z}$ ). A prediction module 501 from the period "k" (k = $n^m$ + 1) by means of the aforesaid contributions or weights deriving from the artificial intelligence module 401 allows predicting a new set of patients (comparable as to the clinical picture to the m of the learning period, but not necessarily in the same number) whose *j* parameters are detected ($0 \leq j \leq n$) the trend of the adherence to therapy. Therefore, while the artificial intelligence module 400 has the health data relative to the previous plurality of patients as input, and is adapted to determine the weight of the parameter and/or the weight of the patient for each parameter and patient of the previous plurality of patients as a function of the health data of the previous plurality of patients, the prediction module 501 refers to the current plurality of patients and is configured to predict the trend of adherence to said therapy by the current plurality of patients as a function of the health data contained in the database DB and as a function of the weight of the parameter and/or of the weight of the patient for each parameter and patient of the previous plurality of patients which are in output from the artificial intelligence module. The learning algorithm 401 provides for the continuous updating of the patient's weight, $w_{i,j}^{t_z}$ , and of the parameter or signal, $p_{i,j}^{t_z}$ .

The contribution of the parameter, $p_{i,j}^{t_z}$ , represents the "weight" of the parameter (within the set of patients treated by the specialist in the reference period) referred to the single reference vital parameter: the greater the weight of the parameter, the more the overall health state of the patients is influenced by the reference parameter (i.e., column by column in the compendium table). By comparing the sum of the compendium by column, an overall objective reference value (the compendium) is obtained that allows to sort the values by column (that is, parameter by parameter) and then to give the specialist a classification of which parameters most influence the group. If this information is known, for example it may be possible for the specialist to focus only on the columns that have a value above a minimum threshold (for example decided by the specialist) and not to use in home monitoring the medical devices that detect the parameters below the threshold (with consequent savings for the NHS).

The contribution or weight of the patient, $w_{i,j}^{t_z}$, on the other hand, represents the weight that the patient has in the group: the sum of the values per row sorts the patients by means of an objective compendium value and allows the specialist to sort his or her priorities of intervention, optimizing his or her time available, focusing, for example, only on patients above a certain critical (minimum) threshold of compendium value (for example decided by the specialist).

In a period of scarcity of resources for the Health Service, the management of priorities through weights, on which the period compendium depends, by row (patient) and by column (parameter), allows the NHS to make choices that make the basket of care opportunities excellent: 1) choosing a smaller number of medical devices to use at home (by fixing the compendium threshold per column); 2) focusing on a limited number of patients and/or prioritizing the intervention on the patients as a function of the compendium threshold per row (assuming that below this threshold however the situation relative to therapeutic compliance is under-control).

The dynamic control of these trends therefore makes it possible to optimize the treatments for the chronic patients in an innovative way.

The database DB of the memory 25 may contain the health data of the current plurality of patients and of the previous plurality of patients or may contain only the health data of the current plurality of patients once the weights at the output from the learning algorithm 401 have been processed.

The learning algorithm 401 is based on the formation of period compendium tables per patient ( $C_i^{t_z}$ ) and per health parameter ( $C_j^{t_z}$ ), distinguishing the contribution or weight of the patient ( $w_{i,j}^{t_z}$ ) from the contribution or weight of the signal or of the parameter ( $p_{i,j}^{t_z}$ ), and on feeding the aforesaid tables with the membership functions received from the devices 100 and stored in the database DB of the server 500.

The period compendium per patient ( $C_i^{t_z}$ ) is the sum of the product of the membership functions $\mu(x_{i,j}^{t_z})$ for the contributions or weights $w_{i,j}^{t_z}$ of the patient:

$$C_i^{t_z} = \sum_{j=1}^{n} \mu(x_{i,j}^{t_z}) \cdot w_{i,j}^{t_z}.$$

The relative table of the period compendium per patient ( $C_i^{t_z}$ ), provided by the module 402 belonging to the module 400, is presented below:

| $x_{i,j}^{t_z}$ | | $x_j^{t_z}$ | | | | | $C_i^{t_z}$ |
|---|---|---|---|---|---|---|---|
| | | $j=1$ | $j=2$ | $j=3$ | ... | $j=n$ | |
| $x_i^{t_z}$ | $i=1$ | $\mu(x_{1,1}^{t_z}) \cdot w_{1,1}^{t_z}$ | $\mu(x_{1,2}^{t_z}) \cdot w_{1,2}^{t_z}$ | $\mu(x_{1,3}^{t_z}) \cdot w_{1,3}^{t_z}$ | ... | $\mu(x_{1,n}^{t_z}) \cdot w_{1,n}^{t_z}$ | $C_{1,j}^{t_z}$ |
| | $i=2$ | $\mu(x_{2,1}^{t_z})$ $\cdot w_{2,1}^{t_z}$ | $\mu(x_{2,2}^{t_z})$ $\cdot w_{2,2}^{t_z}$ | $\mu(x_{2,3}^{t_z})$ $\cdot w_{2,3}^{t_z}$ | ... | $\mu(x_{2,n}^{t_z})$ $\cdot w_{2,n}^{t_z}$ | $C_{2,j}^{t_z}$ |
| | $i=3$ | $\mu(x_{3,1}^{t_z})$ $\cdot w_{3,1}^{t_z}$ | $\mu(x_{3,2}^{t_z})$ $\cdot w_{3,2}^{t_z}$ | $\mu(x_{3,3}^{t_z})$ $\cdot w_{3,3}^{t_z}$ | ... | $\mu(x_{3,n}^{t_z})$ $\cdot w_{3,n}^{t_z}$ | $C_{3,j}^{t_z}$ |
| | ... | ... | ... | ... | ... | ... | ... |
| | $i=m$ | $\mu(x_{m,1}^{t_z})$ $\cdot w_{m,1}^{t_z}$ | $\mu(x_{m,2}^{t_z})$ $\cdot w_{m,2}^{t_z}$ | $\mu(x_{m,3}^{t_z})$ $\cdot w_{m,3}^{t_z}$ | ... | $\mu(x_{m,n}^{t_z})$ $\cdot w_{m,n}^{t_z}$ | $C_{m,j}^{t_z}$ |

Since the membership function has as a maximum value 1, when $x_{i,j}^{t_z}=m_{i,j}^{t_z}$ and since the contribution of the patient has a maximum value of 1, the maximum value of the period compendium per patient is *n*:

$$0 \le C_i^{t_z} \le n.$$

The percentage weight of the single period compendium $C_i^{t_z}$ is indicated as $PC_i^{t_z}$.

$$PC_i^{t_z} = \frac{C_i^{t_z}}{\sum_{i=1}^{m} C_i^{t_z}} \cdot 100.$$

$$0 \le PC_i^{t_z} \le 100.$$

The period compendium per parameter ( $C_j^{t_z}$ ) is the sum of the product of the membership functions $\mu(x_{i,j}^{t_z})$ for the contribution or weight $p_{i,j}^{t_z}$ of the parameter or signal:

$$C_j^{t_z} = \sum_{i=1}^{m} \mu(x_{i,j}^{t_z}) \cdot p_{i,j}^{t_z}.$$

The relative period compendium table per health parameter ( $C_j^{t_z}$ ) is presented below:

| $x_{i,j}^{t_z}$ | | $x_j^{t_z}$ | | | | |
|---|---|---|---|---|---|---|
| | | $j = 1$ | $j = 2$ | $j = 3$ | … | $j = n$ |
| $x_i^{t_z}$ | $i = 1$ | $\mu(x_{1,1}^{t_z}) \cdot p_{1,1}^{t_z}$ | $\mu(x_{1,2}^{t_z}) \cdot p_{1,2}^{t_z}$ | $\mu(x_{1,3}^{t_z}) \cdot p_{1,3}^{t_z}$ | … | $\mu(x_{1,n}^{t_z}) \cdot p_{1,n}^{t_z}$ |
| | $i = 2$ | $\mu(x_{2,1}^{t_z}) \cdot p_{2,1}^{t_z}$ | $\mu(x_{2,2}^{t_z}) \cdot p_{2,2}^{t_z}$ | $\mu(x_{2,3}^{t_z}) \cdot p_{2,3}^{t_z}$ | … | $\mu(x_{2,n}^{t_z}) \cdot p_{2,n}^{t_z}$ |
| | $i = 3$ | $\mu(x_{3,1}^{t_z}) \cdot p_{3,1}^{t_z}$ | $\mu(x_{3,2}^{t_z}) \cdot p_{3,2}^{t_z}$ | $\mu(x_{3,3}^{t_z}) \cdot p_{3,3}^{t_z}$ | … | $\mu(x_{3,n}^{t_z}) \cdot p_{3,n}^{t_z}$ |
| | … | … | … | … | … | … |
| | $i = m$ | $\mu(x_{m,1}^{t_z}) \cdot p_{m,1}^{t_z}$ | $\mu(x_{m,2}^{t_z}) \cdot p_{m,2}^{t_z}$ | $\mu(x_{m,3}^{t_z}) \cdot p_{m,3}^{t_z}$ | … | $\mu(x_{m,n}^{t_z}) \cdot p_{m,n}^{t_z}$ |
| $C_j^{t_z}$ | | $C_{i,1}^{t_z}$ | $C_{i,2}^{t_z}$ | $C_{i,3}^{t_z}$ | … | $C_{i,n}^{t_z}$ |

Since the membership function has as maximum value 1, when $x_{i,j}^{t_z} = m_{i,j}^{t_z}$ and since the contribution of the signal has a maximum value of 1, the maximum value of the period compendium per signal is m:

$$0 \leq C_j^{t_z} \leq m.$$

The percentage weight of the single period compendium $C_j^{t_z}$ is:

$$PC_j^{t_z} = \frac{C_j^{t_z}}{\sum_{j=1}^{n} C_j^{t_z}} \cdot 100.$$

$$0 \leq PC_j^{t_z} \leq 100.$$

As mentioned, the determination of the contribution or weight of the patient ( $w_{i,j}^{t_z}$ ) and of the contribution or weight of the signal or parameter ( $p_{i,j}^{t_z}$ ) is the target of the learning algorithm, the mechanism of which takes place for a set *mxn,* of m patients whose *n* parameters are measured.

Learning begins at the time $t_z$, *with z = 0,* starting from the assumptions that there are no differences between the contribution or weight of the parameters and of the patients:

$$w_{i,j}^{t_z} = \frac{1}{n}$$

$$p_{i,j}^{t_z} = \frac{1}{m}$$

The compendia are calculated:

$$C_j^{t_z} = \sum_{i=1}^{m} \mu\left(x_{i,j}^{t_z}\right) \cdot p_{i,j}^{t_z}$$

$$C_i^{t_z} = \sum_{j=1}^{n} \mu(x_{i,j}^{t_z}) \cdot w_{i,j}^{t_z}$$

$$C_i^{t_z}\left(w_{i,j}^{t_z}=\frac{1}{n}\right) = \sum_{j=1}^{n} \mu(x_{i,j}^{t_z}) \cdot \frac{1}{n}$$

$$C_j^{t_z}\left(p_{i,j}^{t_z}=\frac{1}{m}\right) = \sum_{i=1}^{m} \mu\left(x_{i,j}^{t_z}\right) \cdot \frac{1}{m}.$$

At the time $t_z$ *with* $z = 1$, the following assumptions are made:

$$w_{i,j}^{t_z} = PC_j^{t_{z-1}}$$

$$p_{i,j}^{t_z} = PC_i^{t_{z-1}}$$

The compendia are calculated:

$$C_i^{t_z}\left(w_{i,j}^{t_z}=PC_j^{t_{z-1}}\right) = \sum_{j=1}^{n} \mu(x_{i,j}^{t_z}) \cdot \frac{C_j^{t_{z-1}}}{\sum_{j=1}^{n} C_j^{t_{z-1}}}$$

$$C_j^{t_z}\left(p_{i,j}^{t_z} = PC_i^{t_{z-1}}\right) = \sum_{i=1}^{m} \mu\left(x_{i,j}^{t_z}\right) \cdot \frac{C_i^{t_{z-1}}}{\sum_{i=1}^{m} C_i^{t_{z-1}}}.$$

In the interval of $t_z$ $1 < z < (n^m - 1)$, an iterative process of learning and calculating $C_i^{t_z}$ and $C_j^{t_z}$ is continued, which ends at the time $t_z$, *with* $z = n^m$:

$$C_i^{t_{n^m}} = \sum_{j=1}^{n} \mu(x_{i,j}^{t_{n^m}}) \cdot \frac{C_j^{t_{(n^m-1)}}}{\sum_{j=1}^{n} C_j^{t_{(n^m-1)}}}$$

$$C_j^{t_{n^m}} = \sum_{i=1}^{m} \mu\left(x_{i,j}^{t_{n^m}}\right) \cdot \frac{C_i^{t_{(n^m-1)}}}{\sum_{i=1}^{m} C_i^{t_{(n^m-1)}}}.$$

In the table of the period compendia per health parameter ( $C_j^{t_z}$ ) and per patient ( $C_i^{t_z}$ ), the value of the single box is identical, in both cases; that is, the equivalence is always valid:

$$\mu\left(x_{i,j}^{t_z}\right) \cdot w_{i,j}^{t_z} = \mu\left(x_{i,j}^{t_z}\right) \cdot p_{i,j}^{t_z}, \forall \, pair \, i,j$$

Then, by setting $w_{i,j}^{t_{n^m}} = p_{i,j}^{t_{n^m}}$ , as the target of the learning algorithm 401, and by indicating it as ( $ML_{i,j}^{t_{n^m}}$ ) it is established that this ends at the time $t_z$, where $z = n^m$, when the following final value is calculated:

$$ML_{i,j}^{t_{n^m}} \left(w_{i,j}^{t_{n^m}} = p_{i,j}^{t_{n^m}}\right) = \frac{C_j^{t_{(n^m-1)}}}{\sum_{j=1}^{n} C_j^{t_{(n^m-1)}}} = \frac{C_i^{t_{(n^m-1)}}}{\sum_{i=1}^{m} C_i^{t_{(n^m-1)}}}$$

As mentioned, from the period $k$ [$k = (n^m + 1)$], the results of the artificial intelligence module 401 are distributed to a set of patients $p$, ($p = 1, ..., s$), who are comparable, as to the clinical picture, to the $i$ patients of the learning period ($i = 1, ..., m$); of course, $p$ can also be different from $i$, in total number as well as in type (in the sense that the learning results can be applied to a set of patients having different numerical, with the same pathology and/or degree of morbidity), provided that $j$ parameters are detected. To apply the results of the artificial intelligence module 401 to each new set of patients [$pxj$], from the time $t_k$ a prediction module 501 processes the period compendium matrix by drawing from the contribution $ML_{i,j}^{t_{n^m}}$ according to the following correlation:

$$ML_{p,j}^{t_k} = ML_{i,j}^{t_{n^m}}, \forall \, p = i$$

At the time $t_k$ the sorting of the patients, from $p = 1$ to $p = s$ is defined by the user (preferably the specialist who prescribed home care), according to a subjective priority order; the specialist decides the priority of the order of patients, and the apparatus places $ML_{i,j}^{t_{n^m}}$ in the relative box ($i,j$), determining the following period compendium table of the prediction module 501.

| $x^{t_k}_{p,j}$ | | $x^{t_k}_j$ | | | | |
|---|---|---|---|---|---|---|
| | | $j=1$ | $j=2$ | $j=3$ | ... | $j=n$ |
| $x^{t_k}_p$ | $p=1$ | $\mu(x^{t_k}_{1,1}) \cdot ML^{t_k}_{1,1}$ | $\mu(x^{t_k}_{1,2}) \cdot ML^{t_k}_{1,2}$ | $\mu(x^{t_k}_{1,3}) \cdot ML^{t_k}_{1,3}$ | ... | $\mu(x^{t_k}_{1,n}) \cdot ML^{t_k}_{1,n}$ |
| | $p=2$ | $\mu(x^{t_k}_{2,1}) \cdot ML^{t_k}_{2,1}$ | $\mu(x^{t_k}_{2,2}) \cdot ML^{t_k}_{2,2}$ | $\mu(x^{t_k}_{2,3}) \cdot ML^{t_k}_{2,3}$ | ... | $\mu(x^{t_k}_{2,n}) \cdot ML^{t_k}_{2,n}$ |
| | $p=3$ | $\mu(x^{t_k}_{3,1}) \cdot ML^{t_k}_{3,1}$ | $\mu(x^{t_k}_{3,2}) \cdot ML^{t_k}_{3,2}$ | $\mu(x^{t_z}_{3,3}) \cdot ML^{t_k}_{3,3}$ | ... | $\mu(x^{t_k}_{3,n}) \cdot ML^{t_k}_{3,n}$ |
| | ... | ... | ... | ... | ... | ... |
| | $p=s$ | $\mu(x^{t_k}_{s,1}) \cdot ML^{t_k}_{s,1}$ | $\mu(x^{t_k}_{s,2}) \cdot ML^{t_k}_{s,2}$ | $\mu(x^{t_k}_{s,3}) \cdot ML^{t_k}_{s,3}$ | ... | $\mu(x^{t_k}_{s,n}) \cdot ML^{t_k}_{s,n}$ |

The prediction module 501 then calculates the period compendium $C^{t_k}_{p,j}$ taking care to introduce the specific elements of the new set of patients *p* for each parameter *j*, at the time $t_k$ i.e. the *adherence* and preferably the *correlation* and the *homogeneity*.

Their application allows to adapt the result of the module 401 so as to take into account the peculiarity of each new set of data (identified by a different index, "*j*" vs. "*p*"), as explained below.

The " correlation of the parameter",

$$COV^{t_k}_{p,j} = 1 - \frac{\sum^s_{p=1}\sum^n_{j=1}\left(x^{t_k}_{p,j}-\overline{x^{t_k}_{p,j}}\right)\times\left(C^{t_k}_{p,j}-\overline{C^{t_k}_{p,j}}\right)}{N} \cdot \frac{N}{N-1}$$

indicates how the values ( $x^{t_k}_{p,j}; C^{t_k}_{p,j}$ ) deviate from the average values of the two parameters ( $\overline{x^{t_k}_{p,j}}; \overline{C^{t_k}_{p,j}}$ ) and whether the fluctuations around the average of the two variables are concordant or discordant.

The correlation index of the parameter is comprised between zero and 1:

$$0 < COV^{t_k}_{i,j} \le 1$$

The *adherence of the parameter,*

$$AD^{t_k}_{p,j} = \int_{m^{t_k}_{p,j}-\sigma^{t_k}_{p,j}}^{m^{t_k}_{p,j}+\sigma^{t_k}_{p,j}} \mu\left(x^{t_k}_{p,j}\right) \cdot d\left(x^{t_k}_{p,j}\right)$$

represents the probability that the parameter $x^{t_k}_{p,j}$ assumes a value comprised between $(m^{t_k}_{p,j} - \sigma^{t_k}_{p,j})$ and $(m^{t_k}_{p,j} + \sigma^{t_k}_{p,j})$ , where m is the target value of the parameter imposed by the therapy and $\sigma$ is the possible variation of the parameter as an addition or as a decrease again imposed by the therapy.

The *index of heterogeneity (IO)* gives a measure of the heterogeneity / homogeneity of the statistical distribution of the relative frequencies associated with the *K* modes, i.e. the set of different modes of presentation, of the parameter

$$x_{p,j}^{t_k}(K_{x_{p,j}})$$

.

If the data are distributed heterogeneously over all the K modes of $x_{p,j}^{t_k}$ (i.e., if the modes have similar or, in the case of maximum heterogeneity, equal numerosity), the index is high, conversely, in the case of homogeneous frequency distribution the index is (percentage) rather low.

The index IO is generally defined as follows:

$$IO = 1 - \sum_{i=1}^{k} f_i^2$$

In this case, the index is normalized:

$$IO_{p,j}^{t_k} = \sum_{p=1}^{s} \sum_{j=1}^{n} \left( \frac{Absolute\ Frequency\left(K_{x_{p,j}}\right)}{N} \right)^2 \cdot \frac{N}{N-1},$$

$$(x_{p,j}^{t_k} - \sigma_{p,j}^{t_k}) \leq K_{x_{p,j}} \leq (x_{p,j}^{t_k} + \sigma_{p,j}^{t_k})$$

The Absolute Frequency of $K_{x_{p,j}}$ is the number of times that $x_{p,j}^{t_k}$ is equal to $K_{x_{p,j}}$ regardless of the total number of the samplings of the value. The Relative Frequency is the ratio between the absolute frequency and the number of samplings.

The period compendium $C_{p,j}^{t_k}$ is then calculated by introducing the specific elements of the new set of patients $p$ for each parameter $i$, at the time $t_k$ and their application allows to adapt the result of the learning mechanism so as to take into account the peculiarity of each new set of data (identified by a different index, *"j"* vs. *"p"*).

The calculation of the aforesaid factors allows to find the Therapeutic Compliance ( $CT_{p,j}^{t_k}$ ), i.e. the function of adherence to the therapy by the plurality of patients, which is defined by the product of the aforesaid factors.

$$C_p^{t_k} = \sum_{j=1}^{n} \mu(x_{p,j}^{t_k}) \cdot ML_{p,j}^{t_k}$$

$$C_j^{t_k} = \sum_{p=1}^{s} \mu\left(x_{p,j}^{t_k}\right) \cdot ML_{p,j}^{t_k}.$$

$$CT_{p,j}^{t_k} = C_p^{t_k}(o\ C_j^{t_k}) \cdot AD_{p,j}^{t_k} \cdot IO_{p,j}^{t_k} \cdot COV_{p,j}^{t_k}$$

The specialist can then sort his or her intervention priority as a function of $CT_{p,j}^{t_k}$, choosing between a sorting by patient (thus sorting the values according to the sum of the values per row) or a sorting by parameter (by sorting the values according to the sum of the values by column), in a continuous *input - output* process when the apparatus is connected in the network to the web server 500.

In parallel and for each new plurality of patients who are however comparable, the apparatus calculates, at the time $t_w$, where w = (number of parameters)$^{(number\ of\ patients)}$,

$$ML_{i,j}^{t_w}\left(w_{i,j}^{t_w} = p_{i,j}^{t_w}\right) = \frac{c_j^{t_{(w-1)}}}{\sum_{j=1}^{n} c_j^{t_{(w-1)}}} = \frac{c_i^{t_{(w-1)}}}{\sum_{i=1}^{m} c_i^{t_{(w-1)}}}$$

The specialist for each subsequent (to the first one following the learning) plurality, downloads from the dedicated web server the last $ML_{i,j}^{t_w}$ , calculated as above; this is a seamless process, allowing a snowball effect on the experience.

Continuous tele-monitoring of the vital signs in the home environment can lead to a better knowledge of the prognostic clinical picture of the patients and to an early diagnosis of its possible deterioration, thus facilitating the migration of the treatments (especially of the chronic patients and with the consequent improvement of specialist's confidence) to the home, especially if continuous evaluation of adherence to therapy is guaranteed by recommendation models that are applicable to heterogeneous populations like the present apparatus.

This continuous *input - output* process takes place with the apparatus 1 always connected in the network (with all the devices 100 always connected to the web server 500) and over time it allows to update the position in the table of the parameters and of the patients that depends on the period compendium.

In the process of maximizing the parameter "therapeutic compliance", at the time $t_k$, the web server 500 activates an iterative algorithm 505 belonging to the module 501 that calculates the gradient of the therapeutic compliance function ( $\nabla CT_{p,j}^{t_k}$ ), which has as components the partial derivatives of the function and which describes how the scalar magnitude of the therapeutic compliance varies as a function of its different parameters.

$$\nabla CT_{p,j}^{t_k} = \frac{\partial CT_{p,j}^{t_k}}{\partial C_{p,j}^{t_k}} \cdot \widehat{C_{p,j}^{t_k}} + \frac{\partial CT_{p,j}^{t_k}}{\partial AD_{p,j}^{t_k}} \cdot \widehat{AD_{p,j}^{t_k}} + \frac{\partial CT_{p,j}^{t_k}}{\partial IO_{p,j}^{t_k}} \cdot \widehat{IO_{p,j}^{t_k}} + \frac{\partial CT_{p,j}^{t_k}}{\partial COV_{p,j}^{t_k}} \cdot \widehat{COV_{p,j}^{t_k}}$$

where: $\widehat{C_{p,j}^{t_k}}, \widehat{AD_{p,j}^{t_k}}, \widehat{IO_{p,j}^{t_k}}, \widehat{COV_{p,j}^{t_k}}$ , are the versors along the relative axes.

The directional derivative of the therapeutic compliance function in one of its points represents the numerical value given by the limit of the ratio between the variation it undergoes starting from the point due to a displacement along the direction and sense, identified by the versor with respect to which it is derived and the same displacement with the latter tending to zero and is therefore positive if the therapeutic compliance function is increasing along this sense starting from the point considered, negative or null if not; on the other hand, the directional derivative of the gradient, precisely because of its link with the scalar product, is maximum (and positive) along the versor that identifies it, just as the scalar product of a vector for a versor is maximum and positive when the versor has the direction and sense the vector.

Preferably, the system processes and provides as output the gradient vector field, which is displayed by means of a variable colouring density with the modulus 510.

To indicate the vector field of $CT_{p,j}^{t_k}$ use is made of the concept of chromatic gradient; that is, the gradient $\nabla CT_{p,j}^{t_k}$ is displayed by means of an increasingly intense colouring density to represent gradually higher values assumed by the functions (and consequently, increasing positions in the classification, by patient and by parameter) and which arise from the trend of the gradient.

For example, considering the following table of values of the gradient $\nabla CT_{p,j}^{t_k}$

| $\nabla CT_{p,j}^{t_k}$ | | $x_j^{t_{k+n}}$ | | | | |
|---|---|---|---|---|---|---|
| | | $j = 1$ | $j = 2$ | $j = 3$ | ... | $j = n$ |
| $x_p^{t_k}$ | $p = 1$ | $\nabla CT_{1,1}^{t_k}$ | $\nabla CT_{1,2}^{t_k}$ | $\nabla CT_{1,3}^{t_k}$ | ... | $\nabla CT_{1,n}^{t_k}$ |
| | $p = 2$ | $\nabla CT_{2,1}^{t_k}$ | $\nabla CT_{2,2}^{t_k}$ | $\nabla CT_{2,3}^{t_k}$ | ... | $\nabla CT_{2,n}^{t_k}$ |
| | $p = 3$ | $\nabla CT_{3,1}^{t_k}$ | $\nabla CT_{3,2}^{t_k}$ | $\nabla CT_{3,3}^{t_k}$ | ... | $\nabla CT_{3,n}^{t_k}$ |
| | ... | ... | ... | ... | ... | ... |
| | $p = s$ | $\nabla CT_{s,1}^{t_k}$ | $\nabla CT_{s,2}^{t_k}$ | $\nabla CT_{s,3}^{t_k}$ | ... | $\nabla CT_{s,n}^{t_k}$ |

The graphical representation of the gradient of the therapeutic compliance is the same grey scale table with less intense colouring going from left to right (looking at the table) for the gradient referred to the signal and from top to bottom (always looking at the table) for the gradient referred to the patient:

| $\nabla CT_{p,j}^{t_k}$ | | $x_j^{t_k}$ | | | | |
|---|---|---|---|---|---|---|
| | | $j = 1$ | $j = 2$ | $j = 3$ | ... | $j = n$ |
| $x_p^{t_k}$ | $p = 1$ | $\nabla CT_{1,1}^{t_k}$ | $\nabla CT_{1,2}^{t_k}$ | $\nabla CT_{1,3}^{t_k}$ | ... | $\nabla CT_{1,n}^{t_k}$ |
| | $p = 2$ | $\nabla CT_{2,1}^{t_k}$ | $\nabla CT_{2,2}^{t_k}$ | $\nabla CT_{2,3}^{t_k}$ | ... | $\nabla CT_{2,n}^{t_k}$ |
| | $p = 3$ | $\nabla CT_{3,1}^{t_k}$ | $\nabla CT_{3,2}^{t_k}$ | $\nabla CT_{3,3}^{t_k}$ | ... | $\nabla CT_{3,n}^{t_k}$ |
| | ... | ... | ... | ... | ... | ... |
| | $p = s$ | $\nabla CT_{s,1}^{t_k}$ | $\nabla CT_{s,2}^{t_k}$ | $\nabla CT_{s,3}^{t_k}$ | ... | $\nabla CT_{s,n}^{t_k}$ |
| *Chromatic gradient of the signal* | | $+ \rightarrow -$ | | | | |

| $\nabla CT_{p,j}^{t_k}$ | | $x_j^{t_k}$ | | | | | Chromatic gradient of the patient |
|---|---|---|---|---|---|---|---|
| | | $j = 1$ | $j = 2$ | $j = 3$ | ... | $j = n$ | |
| $x_p^{t_k}$ | $p = 1$ | $\nabla CT_{1,1}^{t_k}$ | $\nabla CT_{1,2}^{t_k}$ | $\nabla CT_{1,3}^{t_k}$ | ... | $\nabla CT_{1,n}^{t_k}$ | $+$ |
| | $p = 2$ | $\nabla CT_{2,1}^{t_k}$ | $\nabla CT_{2,2}^{t_k}$ | $\nabla CT_{2,3}^{t_k}$ | ... | $\nabla CT_{2,n}^{t_k}$ | $\rightarrow$ |
| | $p = 3$ | $\nabla CT_{3,1}^{t_k}$ | $\nabla CT_{3,2}^{t_k}$ | $\nabla CT_{3,3}^{t_k}$ | ... | $\nabla CT_{3,n}^{t_k}$ | $-$ |
| | ... | ... | | | ... | | |
| | $p = s$ | $\nabla CT_{s,1}^{t_k}$ | $\nabla CT_{s,2}^{t_k}$ | $\nabla CT_{s,3}^{t_k}$ | ... | $\nabla CT_{s,n}^{t_k}$ | |

By means of the prediction module 510, the apparatus proposes an innovative system for predicting the trend of the reference parameter as a tendency to its worsening (*FV* -) or improvement (*EV* +) of the reference parameter, at the time (*k* + *n*).

The apparatus 1, in particular the web server 500, and in particular the prediction module 501, for each membership function $\mu\left(x_{p,j}^{t_{k+n}}\right)$ received from the devices 100, at the time $t_{k+n}$ (therefore after learning) processes a square sub-matrix of the input data ( $MC_{p,j}^{t_{k+n}}$ ) positioned south-east of $x_{p,j}^{t_{k+n}}$ , obtained by eliminating the *i - th* row(s) and the *p - th* column(s) (refer to the area in grey in the table below) at the value of the highest compendium per row and/or per column, as a function of the priorities (however sorted) of the specialist (per patient, then per row or per parameter and then per column) with respect to the value of $CT_{p,j}^{t_{k+n}}$ .

| $\mu(x_{p,j}^{t_{k+n}})$ | | $\mu(x_j^{t_{k+n}})$ | | | | |
|---|---|---|---|---|---|---|
| | | $j = 1$ | $j = 2$ | $j = 3$ | ... | $j = n$ |
| $\mu(x_p^{t_{k+n}})$ | $p = 1$ | $\mu(x_{1,1}^{t_{k+n}})$ | $\mu(x_{1,2}^{t_{k+n}})$ | $\mu(x_{1,3}^{t_{k+n}})$ | ... | $\mu(x_{1,n}^{t_{k+n}})$ |
| | $p = 2$ | $\mu(x_{2,1}^{t_{k+n}})$ | $\mu(x_{2,2}^{t_{k+n}})$ | $\mu(x_{2,3}^{t_{k+n}})$ | ... | $\mu(x_{2,n}^{t_{k+n}})$ |
| | $p = 3$ | $\mu(x_{3,1}^{t_{k+n}})$ | $\mu(x_{3,2}^{t_{k+n}})$ | $\mu(x_{3,3}^{t_{k+n}})$ | ... | $\mu(x_{3,n}^{t_{k+n}})$ |
| | ... | ... | ... | ... | ... | ... |
| | $p = s$ | $\mu(x_{s,1}^{t_{k+n}})$ | $\mu(x_{s,2}^{t_{k+n}})$ | $\mu(x_{s,3}^{t_{k+n}})$ | ... | $\mu(x_{s,n}^{t_{k+n}})$ |

For each sub-matrix ( $MC_{p,j}^{t_{k+n}}$ ), the minor ( $M_{p,j}^{t_{k+n}}$ ) is calculated, of which account is taken for the absolute value $\left|M_{p,j}^{t_{k+n}}\right|$ . The sub-matrix $MC_{p,j}^{t_{k+n}}$ then considers all the data of the matrix with the exception of the first column(s) and of the first row(s), sorted by increasing or decreasing values (according to the preferences of the specialist, who can sort his or her preferences by patient/row or by parameter/column).

The comparison between the variation of the therapeutic compliance or adherence function ( $CT_p^{t_{k+n}}$ ) and the variation of the minor of the sub-matrix of the input data ( $MC_{p,j}^{t_{k+n}}$ ) over time is the result of the processing that the prediction module 501 performs by exposing in the table a tendency (EV) to worsening [(for each result less than zero of (EV-)] or to improvement [in the opposite case, in which it is greater than zero (EV+)], of the reference parameter; therefore, to the time (*k* + *n*) the following rule applies:

*IF*

$$\frac{CT_{p,j}^{t_{k+n}} - CT_{p,j}^{t_{k+(n-1)}}}{CT_{p,j}^{t_{k+(n-1)}}} > \frac{\left|MC_{p,j}^{t_{k+n}}\right| - \left|MC_{p,j}^{t_{k+(n-1)}}\right|}{MC_{p,j}^{t_{k+(n-1)}}}$$

*AND*

$$\frac{\mu(x_{p,j}^{t_{k+n}}) - \mu(x_{p,j}^{t_{k+(n-1)}})}{\mu(x_{p,j}^{t_{k+(n-1)}})} > 0$$

*THEN*

$$EV\left(\frac{\mu(x_{p,j}^{t_{k+(n+1)}}) - \mu(x_{p,j}^{t_{k+n}})}{\mu(x_{p,j}^{t_{k+n}})}\right) > 0.$$

Preferably, in accordance with a variant of the embodiment of the present invention, the apparatus comprises a module 600 for displaying the data in output from the prediction module 501, preferably connected with the Data Base DB, and provided with a user interface 601. The module 600, used by the specialist, allows the latter to be provided with an immediate decision-making tool, in a continuous input-output process when the apparatus is connected to the web server 500.

Through the interface 601, at a request from the specialist, the module 600, by drawing on the tables prepared by the apparatus and presented so far, answers by sending an updated list of patients and/or of parameters, preferably sorted in descending order and preferably according to the resultants of the module 501 and by period; the length of this list is preferably defined by the specialist, preferably as a function of his or her needs in programming his or her own resources for taking charge of the patient with chronic pathology.

For example, the specialist can query the system, via the interface 601, in order to obtain the first ten patients who need a priority of intervention as a function of the period compendium value.

In the same way, for example, the specialist can query the system to provide the first three vital parameters that have priority for evaluation.

The choice of the number of the patients to give attention to depends preferably on the time available for the service, for each period, while the choice of the number of parameters to be measured depends preferably on the resources available for the purchase and/or the management of the input machinery 100.

The module 600 answers to this request, by drawing from the tables and/or in any case from the predictive values of the period processed by the system.

Preferably, the interface 601 used by the specialist therefore provides for the definition of the number of patients and/or of the number of parameters, as methods for filtering the query at the module 600, in order to preferably obtain as an answer the simple list of the patients and/or of the parameters to be prioritised, by drawing on the prediction algorithms, the tables and in general, the processings of the apparatus, which are preferably not presented in their completeness to the specialist, preferably for reasons of display economy and immediacy of understanding.

In a period of scarcity of resources for healthcare, the management of the priorities of the patients and of the parameters by means of the present apparatus can allow the specialist to make choices that optimise the basket of the treatment opportunities, by choosing, with the same adherence to therapy, a smaller number of medical devices to be used at home (and thus saving money) and/or by focusing on a limited number of patients, prioritising intervention on the patients who need it most, also in a prognostic logic, since the dynamic control of these trends makes it possible to optimise the cures for the chronic patients in an innovative way, also thanks to an objective evaluation of the therapeutic compliance, preferably with predictive value.

The invention thus conceived is susceptible to several modifications and variations, all falling within the scope of the inventive concept.

Moreover, all details can be replaced by other technically equivalent elements.

In practice, the materials used, as well as the contingent shapes and sizes, can be whatever according to the requirements without for this reason departing from the scope of protection of the following claims.

## Claims

1. Apparatus (1) for monitoring the parameters of a plurality of patients and for predicting the trend of the adherence by the plurality of patients to a therapy set by a specialist, said apparatus comprising:

   - a plurality of devices (100) adapted to collect parameters on the health of the plurality of patients relative to said therapy and to process health data in relation to said parameters,
   - a central unit (500) adapted to receive health data of the plurality of patients from said plurality of devices; said central unit comprising
   - a memory (25) in which a database (DB) containing said health data on the plurality of patients is installed,
   - an artificial intelligence module (400) connected with said database and comprising at least one learning algorithm (401), said artificial intelligence module being adapted to determine the weight of the parameter and/or the weight of the patient for each parameter and patient of the previous plurality of patients as a function of the health data of the previous plurality of patients,
   - a prediction module (501) connected with said database and configured to predict the trend of the adherence to said therapy by the current plurality of patients as a function of the health data contained in the database and as a function of the weight of the parameter and/or of the weight of the patient for each parameter and patient of the previous plurality of patients in output from the artificial intelligence module.

2. Apparatus (1) according to claim 1, **characterized in that** each device of said plurality of devices comprises an executive software (FL) installed in said memory (SD), said software operating in accordance with a fuzzy logic in which basic rules (201) are defined and membership functions ( $\mu\left(x_{i,t}^{t_z}\right), \mu\left(x_{i,f}^{t_z}\right), \mu\left(x_{i,s}^{t_z}\right), \mu\left(x_{i,b}^{t_z}\right), \mu\left(x_{i,p}^{t_z}\right), \mu\left(x_{i,d}^{t_z}\right), \mu\left(x_{i,w}^{t_z}\right)$ ) for all the parameters on the health of the patients in input are calculated, each device being adapted to output the membership functions relative to the parameters on health in input and which represent said health data.

3. Apparatus (1) according to claim 2, **characterized in that** said parameters on health come from external sensors.

4. Apparatus (1) according to claim 2, **characterized in that** said parameters on health comprise values on the condition of the patient that can be entered from the outside and memorized in said memory (SD), the membership function relative to each of said values on the condition of the patient assuming a value 1 or 0 depending on the presence or absence of the value on the condition of the patient.

5. Apparatus (1) according to claim 2, **characterized in that** said learning algorithm (401) comprises the formation of period compendium tables per patient ( $C_i^{t_z}$ ) and/or per health parameter ( $C_j^{t_z}$ ), distinguishing the contribution or weight of the patient ( $w_{i,j}^{t_z}$ ) from the contribution or weight of the signal or of the parameter ( $p_{i,j}^{t_z}$ ), and on the nutrition of the aforesaid tables with the membership functions received from said plurality of devices (100).

6. Apparatus (1) according to claim 5, **characterized in that** said prediction module (501) processes a further period compendium ( $C_{p,j}^{t_k}$ ) as a function of the weight of the patient ( $w_{i,j}^{t_z}$ ) and/or of the weight of the parameter ( $p_{i,j}^{t_z}$ ), said prediction module being configured to process a function of adherence ( $CT_{p,j}^{t_k}$ ) to said therapy by the current plurality of patients as a function of said further period compendium for at least one specific adherence element ( $AD_{p,j}^{t_k}$ ).

7. Apparatus (1) according to claim 6, **characterized in that** said function of adherence ( $CT_{p,j}^{t_k}$ ) to said therapy by the plurality of patients is processed also as a function of a specific correlation element ( $COV_{p,j}^{t_k}$ ) and a specific homogeneity element ( $IO_{p,j}^{t_k}$ ).

8. Apparatus (1) according to claim 6 or 7, **characterized in that** said prediction module comprises an iterative algorithm (505) configured to process the gradient of the function of adherence to therapy by said current plurality of patients ( $\nabla CT_{p,j}^{t_k}$ ) in order to determine the trend of adherence to said therapy by the current plurality of patients.

9. Apparatus (1) according to claim 8, **characterized in that** said prediction module comprises a further module (510) configured to display the vector field of the gradient by means of a variable colouring density.

10. Apparatus (1) according to claim 9, **characterized in that** said prediction module (501), for each membership function ( $\mu\left(x_{p,j}^{t_{k+n}}\right)$ ) received from the plurality of devices (100) at the time $t_{k+n}$ is adapted to process a square sub-matrix ( $MC_{p,j}^{t_{k+n}}$ ) and to calculate the minor thereof ( $M_{p,j}^{t_{k+n}}$ ) of which the absolute value is considered ( $\left| M_{p,j}^{t_{k+n}} \right|$ )said prediction module being adapted to make the comparison between the variation of the adherence function ( $CT_{p,j}^{t_{k+n}}$ ) and the variation of said minor of the sub-matrix ( $MC_{p,j}^{t_{k+n}}$ ) over time, said comparison being interpreted as a tendency (EV) to the worsening or to the improvement of said function of adherence to the therapy by the plurality of patients.

11. Apparatus (1) according to any one of the preceding claims, **characterized in that** it comprises a display module (600) connected to said prediction module (501) and adapted to issue a priority list of patients on whom to intervene, said display module being provided with a user interface with which the user can define the number of the patients on the list that said display module must issue.

FIG.1

# FIG.3

# FIG.4

EP 4 411 747 A1

FIG.5

Europäisches
Patentamt

European
Patent Office

Office européen
des brevets

# EUROPEAN SEARCH REPORT

Application Number

EP 24 15 5710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/391081 A1 (GOLDNER DANIEL R [US] ET AL) 16 December 2021 (2021-12-16) * figure 1 * * paragraph [0004] * * paragraph [0028] * * paragraph [0036] – paragraph [0037] * * paragraph [0132] – paragraph [0135] * ----- | 1-11 | INV. G16H20/60 G16H50/70 |

TECHNICAL FIELDS
SEARCHED      (IPC)

G16H

The present search report has been drawn up for all claims

| Place of search | Date of completion of the search | Examiner |
|---|---|---|
| Munich | 16 May 2024 | Gardiner, Alexander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.........................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

**EP 24 15 5710**

This annex lists the patent family members relating to the patent documents cited in the above-mentioned European search report.
The members are as contained in the European Patent Office EDP file on
The European Patent Office is in no way liable for these particulars which are merely given for the purpose of information.

**16-05-2024**

| Patent document cited in search report | Publication date | Patent family member(s) | Publication date |
|---|---|---|---|
| US 2021391081 A1 | 16-12-2021 | US 2021391081 A1 | 16-12-2021 |
| | | WO 2021247922 A1 | 09-12-2021 |

EPO FORM P0459

For more details about this annex : see Official Journal of the European Patent Office, No. 12/82